# FASCICULE DE BREVET EUROPEEN

(11) **EP 2 149 365 B1**
(45) Date de publication et mention de la délivrance du brevet: **27.01.2016**
(21) Numéro de dépôt: 09151421.6
(22) Date de dépôt: 27.01.2009
(51) Int. Cl.: A61K 8/81, A61Q 5/06, A61K 8/73, A61K 8/87, A61Q 5/12

(54) **Composition cosmétique comprenant un copolymère vinylformamide / vinylformamine et un polymère épaississant**
Kosmetische Zusammensetzung enthalted ein Kopolymer aus Vinylformamid/Vinylformamine und ein verdickendes Polymer
Cosmetic composition comprising a copolymer of vinylformamid/vinylformamine and a thickening polymer

(30) Priorité: 31.01.2008 FR 0850607
(43) Date de publication de la demande: 03.02.2010
(73) Titulaire: L'Oréal, 75008 Paris (FR)
(72) Inventeur: Mathonneau, Estelle, 75010 PARIS (FR); Samain, Henri, 91570 BIEVRES (FR); Benabdillah, Katarina, 30140 Saint Jean de Pin (FR)
(74) Mandataire: Bourdeau, Françoise

(56) Documents cités:
- EP-A- 1 779 894
- WO-A-02/15854
- WO-A-2007/003784
- DE-A1-102005 014 293
- JP-A- 2002 255 756
- US-A- 5 632 977
- US-A1- 2005 245 684
- US-A1- 2006 286 057
- US-A1- 2007 110 690

## Description

La présente invention est relative à une composition cosmétique comprenant, dans un milieu cosmétiquement acceptable, un ou plusieurs copolymères vinylformamide / vinylformamine et un ou plusieurs polymères épaississants.

La présente invention concerne également un procédé de traitement cosmétique des cheveux, notamment un procédé de fixation et/ou de mise en forme des cheveux mettant en oeuvre la composition précitée.

La présente invention concerne enfin une utilisation de cette composition pour le traitement cosmétique capillaire, et en particulier pour le coiffage des cheveux, c'est-à-dire la mise en forme et/ou la fixation de la coiffure.

Les produits de coiffage sont habituellement utilisés pour construire, structurer la coiffure et lui apporter une tenue durable. Ils se présentent usuellement sous forme de lotions, gels, mousses, crèmes, sprays, etc. Les compositions correspondantes comprennent généralement un ou plusieurs polymères filmogènes ou « polymères fixants », dans un milieu cosmétiquement acceptable. Ces polymères permettent la formation d'un film gainant sur les cheveux, assurant ainsi le maintien de la coiffure.

Toutefois, les films de polymère fixant ainsi formés présentent l'inconvénient d'être relativement friables, ce qui limite dans le temps la tenue de la coiffure, et engendre la formation sur les cheveux de résidus inesthétiques.

Ainsi, les produits de coiffage classiques conduisent à une fixation de la coiffure et des effets coiffants qui s'estompent progressivement au cours du temps. En particulier, lorsque le produit est appliqué le matin, les effets coiffants s'estompent au fur et à mesure dans la journée. Le lendemain, les effets coiffants sont faibles, voire inexistants.

Pour remédier à ce problème, il est connu d'incorporer dans les produits de coiffage des polymères à très haut pouvoir fixant, et/ou d'augmenter la concentration en polymère fixant. Toutefois, l'emploi de tels produits extrêmement fixants entraine un certain nombre d'inconvénients. En particulier ces produits conduisent à un toucher sec et rugueux des cheveux et sont difficiles à éliminer au shampooing.

Il existe donc un besoin pour des compositions capillaires permettant d'obtenir une fixation durable de la coiffure, avec des effets coiffants persistant toute la journée voire plusieurs jours, tout en s'éliminant facilement au shampooing et en procurant un toucher cosmétique agréable, et notamment un toucher lisse.

La demande de brevet internationale WO 96/03969 décrit d'une manière générale des compositions cosmétiques destinées à la fixation et/ou au conditionnement des cheveux, qui comprennent un homopolymère de vinylformamide ou un copolymère de vinylformamide et d'un ou plusieurs autres monomères vinyliques, en association avec au moins un ingrédient choisi parmi les agents conditionnants, les agents émulsifiants, les tensioactifs, les modificateurs de viscosité, les agents gélifiants, les agents opacifiants, les agents stabilisants, les conservateurs, les agents séquestrants, les agents chélatants, les agents nacrants, les agents clarifiants, les parfums, les colorants, les agents propulseurs, les solvants organiques et l'eau.

Par ailleurs, le brevet américain US 4 421 602 décrit des copolymères vinylformamide / vinylformamine, leur préparation et leur utilisation dans l'industrie panetière pour améliorer la rétention, le taux d'écoulement, et la floculation.

Le document JP-A-2002 255756 décrit des compositions des fixations à base de copolymère d'alcool polyvinylique et polyvinylamine dont certain comprennent des unités polyvinylformamide.

La Demanderesse a maintenant découvert que, de manière surprenante, l'association d'un copolymère vinylformamide / vinylformamine avec un polymère épaississant dans une composition cosmétique non lavante (c'est-à-dire à faible teneur en tensioactifs anioniques et non ioniques, voire exempte de tels tensioactifs) permettait d'obtenir une composition cosmétique capillaire apportant des propriétés de coiffage améliorées. Notamment, une telle association permet d'obtenir des produits de coiffage procurant une fixation durable de la coiffure, tout en s'éliminant facilement au lavage et en procurant un toucher cosmétique agréable des cheveux après le shampooing.

La présente invention permet ainsi de préparer des produits de coiffage procurant des degrés de fixation très élevés, une très longue tenue de la coiffure au cours du temps, et une bonne résistance de celle-ci aux sollicitations mécaniques. Après le shampooing, le toucher des cheveux est particulièrement doux, et les cheveux sont déliés.

La présente invention a donc pour objet une composition cosmétique comprenant, dans un milieu cosmétiquement acceptable :
- un ou plusieurs copolymères vinylformamide / vinylformamine comprenant :
   de 20 à 40 % en moles de motifs de formule suivante A : 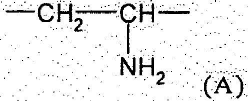
   et de 60 à 80 % en moles de motifs de formule suivante B: 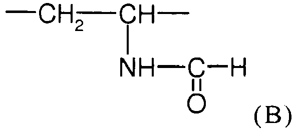
   - un ou plusieurs polymères épaississants associatifs différents des copolymères vinylformamide / vinylformamine. De surcroit les polymères vinylformamide / vinylformanine sont constitués uniquement de motifs A et de motifs B.

Avantageusement, cette composition content moins de 5% en poids au total de tensioactifs anioniques et de tensioactifs non ioniques.

Les compositions selon l'invention permettent en particulier de réaliser des coiffures mèches par mèches notamment sur des cheveux courts, lesquelles coiffures résistent particulièrement bien aux sollicitations mécaniques et présentent une fixation souple ou dure selon les concentrations du copolymère vinylformamide / vinylformamine et de polymère épaississant.

D'autres objets, caractéristiques, aspects et avantages de l'invention apparaîtront encore plus clairement à la lecture de la description et des exemples qui suivent.

Selon l'invention, la composition cosmétique comprend, dans un milieu cosmétiquement acceptable, un ou plusieurs copolymères vinylformamide / vinylformamine, et un ou plusieurs polymères épaississants différents des copolymères vinylformamide / vinylformamine tel que défini ci-dessus.

Par "milieu cosmétiquement acceptable", on entend un milieu compatible avec les matières kératiniques et en particulier les cheveux.

De préférence, le milieu cosmétiquement acceptable comprend de l'eau et/ou un ou plusieurs solvants cosmétiquement acceptables choisis parmi les alcools inférieurs en C₁-C₄, tels que l'éthanol, l'isopropanol, le tertio-butanol ou le n-butanol ; les polyols comme le propylèneglycol ; les éthers de polyols ; les alcanes en C₅-C₁₀ ; les cétones en C₃₋₄ comme l'acétone et la méthyléthylcétone ; les acétates d'alkyle en C₁-C₄ comme l'acétate de méthyle, l'acétate d'éthyle et l'acétate de butyle ; le diméthoxyéthane, le diéthoxyéthane ; et leurs mélanges.

### Copolyméres vinylformamide / vinylformamine

Le ou les copolymères vinylformamide / vinylformamine utilisables dans les compositions selon l'invention comprennent de 20 à 40% en moles de motif de formule A.

Le ou les copolymères vinylformamide / vinylformamine selon l'invention comprennent de 60 à 80% en moles de motif de formule B

Les copolymères selon l'invention peuvent être obtenus par exemple par hydrolyse partielle de polyvinylformamide. Cette hydrolyse peut se faire en milieu acide ou basique.

Le ou les copolymères vinylformamide / vinylformamine selon l'invention sont constitués uniquement de motifs de formule A et de motifs de formule B.

La masse moléculaire moyenne en poids dudit copolymère, mesurée par diffraction de la lumière, peut varier 10.000 à 30.000.000 g/mole, de préférence de 40.000 à 1.000.000 et plus particulièrement de 100.000 à 500.000 g/mole.

La densité de charge cationique dudit copolymère peut varier de 2 meq/g à 20 meq/g, de préférence de 2,5 à 15 et plus particulièrement de 3,5 à 10 meq/g.

A titre d'exemple de copolymères vinylformamide / vinylformamine utilisables dans les compositions selon l'invention, citons entre autres les produits commercialisés sous la dénomination LUPAMIN par la société BASF, tels que par exemple, et de manière non limitative, les produits proposés sous la dénomination LUPAMIN 9030.

Le ou les copolymères vinylformamide / vinylformamine sont présents dans les compositions selon l'invention dans des proportions allant, de préférence, de 0,1 à 25 % en poids, plus préférentiellement de 0,5 à 20 % en poids et plus particulièrement de 1 à 10 % en poids par rapport au poids total de la composition.

### Polymères épaississants

Au sens de la présente invention, on entend par polymère épaississant, un polymère qui introduit à 1% dans une solution aqueuse pure ou hydroalcoolique à 30 % d'éthanol, et à pH = 7, permet d'atteindre une viscosité d'au moins 100 cps, de préférence au moins 500 cps, à 25°C et à un taux de cisaillement de 1s⁻¹. Cette viscosité peut être mesurée à l'aide d'un viscosimètre cône/plan (Rhéomètre Haake R600 ou analogue).

De préférence, ces polymères augmentent par leur présence la viscosité des compositions dans lesquelles ils sont introduits d'au moins 50 cps, de préférence 200cps, à 25°C, et à un taux de cisaillement de 1s⁻¹.

Les polymères épaississants peuvent être des polymères ioniques ou non.

La composition selon l'invention comprend un ou plusieurs polymères épaississants associatifs. Elle peut en outre éventuellement comprendre un ou plusieurs polymères épaississants non associatifs.

En ce qui concerne les polymères épaississants non associatifs, il est tout d'abord rappelé qu'au sens de la présente invention, les polymères épaississants non associatifs sont des polymères épaississants ne contenant pas de chaîne grasse en C₁₀-C₃₀.

Parmi les polymères épaississants non associatifs présents, on peut citer les homopolymères ou copolymères d'acide acrylique ou méthacryliques réticulés, homopolymères réticulés d'acide 2-acrylamido-2-méthyl-propane sulfonique et leurs copolymères réticulés d'acrylamide, les homopolymères d'acrylate d'ammonium ou les copolymères d'acrylate d'ammonium et d'acrylamide, les gommes de guar non ioniques, les gommes de biopolysaccharides d'origine microbienne, les gommes issues d'exudats végétaux, les celluloses en particulier les hydroxypropyl- ou carboxyméthyl- celluloses ; les pectines et les alginates, seuls ou en mélanges.

Une première famille de polymères épaississants non associatifs convenable est représentée par les homopolymères d'acide acrylique réticulés.

Parmi les homopolymères de ce type, on peut citer ceux réticulés par un éther allylique d'alcool de la série du sucre, comme par exemple les produits vendus sous les noms CARBOPOLS 980, 981, 954, 2984 et 5984 par la société NOVEON ou les produits vendus sous les noms SYNTHALEN M et SYNTHALEN K par la société 3 VSA.

Les polymères épaississants non associatifs peuvent être aussi des copolymères d'acide (méth)acryliques réticulés tels que le polymère vendu sous la dénomination AQUA SF1 par la société NOVEON.

Les polymères épaississants non associatifs peuvent être choisis parmi les homopolymères réticulés d'acide 2-acrylamido-2-méthylpropane sulfonique et leurs copolymères réticulés d'acrylamide.

En ce qui concerne ces homopolymères et copolymères, qui peuvent être partiellement ou totalement neutralisés, on peut citer les polymères comprenant de 90 à 99,9% en poids, par rapport au poids total du polymère, de motifs de formule (j) suivante : 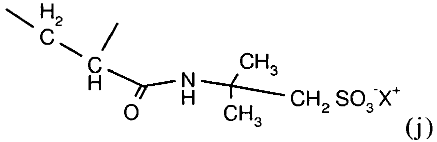 dans laquelle X+ désigne un cation ou un mélange de cations, ou un proton.

Plus particulièrement les cations sont choisis parmi les métaux alcalins (comme le sodium, le potassium), les ions ammonium substitués ou non par un à trois radicaux alkyle, identiques ou différents, comprenant 1 à 6 atomes de carbone, éventuellement porteur d'au moins un radical hydroxyle, les cations dérivant de la N-méthyl-glucamine, d'acides aminés basiques comme l'arginine et la lysine. De préférence, le cation est un ion ammonium ou sodium.

Par ailleurs, le polymère comprend de 0,01 à 10% en poids, par rapport au poids total du polymère, de motifs réticulants provenant d'au moins un monomère ayant au moins deux insaturation éthyléniques (double liaison carbone-carbone).

Les monomères de réticulation ayant au moins deux insaturations éthyléniques sont choisis par exemple parmi l'éther diallylique, le triallylcyanurate, le diallylmaléate, le (méth)acrylate d'allyle, le dipropylèneglycol-diallyléther, les polyglycol-diallyléthers, le triéthylèneglycol-divinyléther, l'hydroquinone-diallyl-éther, le tétrallyl-oxéthanoyle, le di(méth)acrylate de tétra- ou di-éthylèneglycol, la triallylamine, la tétraallyléthylènediamine le triméthylolpropane-diallyléther, le triméthylolpropane triacrylate, le méthylène-bis(méth)acrylamide ou le divinylbenzène, les éthers allyliques d'alcools de la série des sucres, ou d'autres allyl- ou vinyl-éthers d'alcools polyfonctionnels, ainsi que les esters allyliques des dérivés de l'acide phosphorique et/ou vinylphosphonique, ou les mélanges de ces composés.

Pour plus de détail au sujet de ces polymères, on pourra se reporter au document EP 815828.

Parmi les copolymères réticulés d'acide 2-acrylamido-2-méthyl-propane sulfonique et d'acrylamide partiellement ou totalement neutralisés, on peut citer en particulier le produit décrit dans l'exemple 1 du document EP 503 853 et l'on pourra se reporter à ce document pour ce qui a trait à ces polymères.

La composition peut de même comprendre, à titre de polymères épaississants non associatifs, les homopolymères d'acrylate d'ammonium ou les copolymères d'acrylate d'ammonium et d'acrylamide.

A titre d'exemples d'homopolymères d'acrylate d'ammonium, on peut citer le produit vendu sous le nom MICROSAP PAS 5193 par la société HOECHST. Parmi les copolymères d'acrylate d'ammonium et d'acrylamide, on peut citer le produit vendu sous le nom BOZEPOL C NOUVEAU ou le produit PAS 5193 vendus par la société HOECHST. On pourra notamment se référer aux documents FR 2 416 723, US 2798053 et US 2923692 pour ce qui a trait à la description et à la préparation de tels composés.

La composition peut aussi comprendre des homopolymères de diméthylaminoéthyl-méthacrylate quaternisé par le chlorure de méthyle ou les copolymères de diméthylamino-éthylméthacrylate quaternisé par le chlorure de méthyle et d'acrylamide.

Parmi les homopolymères de ce type, on peut citer les produits vendus sous les noms SALCARE SC95 et SALCARE SC96 par la société CIBA. Parmi les copolymères de cette famille, on peut citer le produit SALCARE SC92 vendu par CIBA ou le produit PAS 5194 vendu par HOECHST. Ces polymères sont notamment décrits et préparés dans le document EP 395282 auquel on pourra se référer.

A titre de polymères épaississants non associatifs, on peut citer des gommes de guar non ioniques, comme par exemple les gommes de guar non ioniques non modifiées vendues sous la dénomination VIDOGUM GH 175 par la société UNIPECTINE et sous la dénomination JAGUAR C par la société MEYHALL.

Les gommes de guar non ioniques utilisables sont de préférence modifiées par des groupements hydroxyalkyle en C₁-C₆. Parmi les groupements hydroxyalkyle, on peut mentionner à titre d'exemple, les groupements hydroxyméthyle, hydroxyéthyle, hydroxypropyle et hydroxybutyle.

Ces gommes dé guar sont bien connues de l'état de la technique et peuvent par exemple être préparées en faisant réagir des oxydes d'alcènes correspondants, tels que par exemple des oxydes de propylène, avec la gomme de guar, de façon à obtenir une gomme de guar modifiée par des groupements hydroxypropyle.

Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2.

De telles gommes de guar non ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société MEYHALL ou sous la dénomination GALACTASOL 4H4FD2 par la société AQUALON.

A titre de polymères épaississants non associatifs convenables, on peut aussi mentionner les gommes de biopolysaccharides d'origine microbienne telles que les gommes de scléroglucane ou de xanthane.

Conviennent aussi les gommes issues d'exudats végétaux, telles que les gommes arabiques, gommes Ghatti, gommes Karaya et Tragacanthe ; les celluloses, en particulier les hydroxypropyl- ou carboxyméthyl- celluloses ; les pectines et les alginates.

Ces polymères sont bien connus de l'homme de l'art et sont notamment décrits dans l'ouvrage de Robert L. DAVIDSON intitulé "Handbook of Water soluble gums and resins" édité chez Mc Graw Hill Book Company (1980).

Parmi les agents épaississants, pour l'invention, on utilise les systèmes épaississants à base de polymères associatifs bien connus de l'homme de l'art et notamment de nature non ionique, anionique, cationique ou amphotère.

Il est rappelé que les polymères associatifs sont des polymères hydrophiles capables, dans un milieu aqueux, de s'associer réversiblement entre eux ou avec d'autres molécules.

Leur structure chimique comprend plus particulièrement au moins une zone hydrophile et au moins une zone hydrophobe.

Par groupement hydrophobe, on entend un radical ou polymère à chaîne hydrocarbonée, saturée ou non, linéaire ou ramifiée, comprenant au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel. A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Parmi les polymères associatifs de type anionique, on peut citer :
- (I) ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (I) suivante :

   CH₂ = C R' CH₂ O Bₙ R (I)

   dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (I) plus particulièrement préféré est un motif dans lequel R' désigne H, n est égal à 10, et R désigne un radical stéaryl (C₁₈).

Des polymères associatifs anioniques de ce type sont décrits et préparés, selon un procédé de polymérisation en émulsion, dans le brevet EP-0 216 479.

Parmi ces polymères associatifs anioniques, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (I), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société CIBA sous les dénominations SALCARE SC80® et SALCARE SC90® qui sont des émulsions aqueuses à 30% d'un terpolymère réticulé d'acide méthacrylique, d'acrylate d'éthyle et de steareth-10-allyl éther (40/50/10).
- (II) ceux comportant au moins un motif hydrophile de type acide carboxylique insaturé oléfinique, et au moins un motif hydrophobe de type ester d'alkyl (C₁₀-C₃₀) d'acide carboxylique insaturé.

De préférence, ces polymères sont choisis parmi ceux dont le motif hydrophile de type acide carboxylique insaturé oléfinique correspond au monomère de formule (II) suivante : 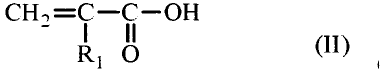 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (III) suivante 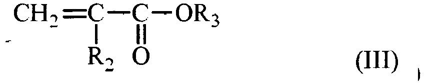 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.

Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères anioniques de ce type sont par exemple décrits et préparés, selon les brevets US-3 915 921 et 4 509 949.

Parmi ce type de polymères associatifs anioniques, on utilisera plus particulièrement des polymères formés à partir d'un mélange de monomères comprenant :
essentiellement de l'acide acrylique,
un ester de formule (III) décrite ci-dessus et dans laquelle R₂ désigne H ou CH₃, R₃ désignant un radical alkyle ayant de 12 à 22 atomes de carbone, et
   (iii) un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères associatifs anioniques, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précédemment.

Parmi lesdits polymères ci-dessus, on préfère tout particulièrement selon la présente invention, les produits vendus par la société GOODRICH sous les dénominations commerciales PEMULEN TRI®, PEMULEN TR2®, CARBOPOL 1382®, et encore plus préférentiellement le PEMULEN TR1®, et le produit vendu par la société S.E.P.P.I.C. sous la dénomination COATEX SX®.

On peut aussi citer les polymères qui, outre les monomères de formule (II) et de formule (III) contiennent un ou plusieurs autres monomères. Ce monomère additionnel peut être notamment un vinyllactame et en particulier la vinylpyrrolidone.

Comme exemple de polymère, on peut citer le terpolymère acide acrylique/méthacrylate de lauryle/vinylpyrrolidone commercialisé sous l'appelation Acrylidone LM par la Société ISP.
- (III) les terpolymères d'anhydride maléique/α-oléfine en C₃₀-C₃₈/ maléate d'alkyle tel que le produit (copolymère anhydride maléique/α-oléfine en C₃₀-C₃₈/maléate d'isopropyle) vendu sous le nom PERFORMA V 1608® par la société NEWPHASE TECHNOLOGIES.
- (IV) les terpolymères acryliques comprenant :
   (a) environ 20% à 70% en poids d'un acide carboxylique à insaturation α,β-monoéthylénique,
   (b) environ 20 à 80% en poids d'un monomère à insaturation α,β-monoéthylénique non-tensio-actif différent de (a),
   (c) environ 0,5 à 60% en poids d'un mono-uréthane non-ionique qui est le produit de réaction d'un tensio-actif monohydrique avec un monoisocyanate à insaturation monoéthylénique,
   tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (40OE) en dispersion aqueuse à 25%.
- (V) les copolymères comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C₁-C₄.

A titre d'exemple de ce type de composé on peut citer l'ACULYN 22® vendu par la société ROHM et HAAS, qui est un terpolymère acide méthacrylique/acrylate d'éthyle/méthacrylate de stéaryle oxyalkyléné.

Parmi les polymères associatifs de type cationique, on peut citer :
- (I) les polyuréthanes associatifs cationiques dont la famille a été décrite par la demanderesse dans la demande de brevet français N° 0009609; elle peut être représentée par la formule générale (IV) suivante :

   R-X-(P)n-[L-(Y)m]r-L'-(P')p-X'-R' (IV)

   dans laquelle :
   R et R', identiques ou différents, représentent un groupement hydrophobe ou un atome d'hydrogène ;
   X et X', identiques ou différents, représentent un groupement comportant une fonction amine portant ou non un groupement hydrophobe, ou encore le groupement L" ;
   L, L' et L", identiques ou différents, représentent un groupement dérivé d'un diisocyanate ;
   P et P', identiques ou différents, représentent un groupement comportant une fonction amine portant ou non un groupement hydrophobe ;
   Y représente un groupement hydrophile ;
   r est un nombre entier compris entre 1 et 100, de préférence entre 1 et 50 et en particulier entre 1 et 25,
   n, m, et p valent chacun indépendamment des autres entre 0 et 1000 ;
   la molécule contenant au moins une fonction amine protonée ou quaternisée et au moins un groupement hydrophobe.

Dans un mode de réalisation préféré de ces polyuréthanes, les seuls groupements hydrophobes sont les groupes R et R' aux extrémités de chaîne.

Une famille préférée de polyuréthanes associatifs cationiques est celle correspondant à la formule (IV) décrite ci-dessus et dans laquelle :
R et R' représentent tous les deux indépendamment un groupement hydrophobe,
X, X' représentent chacun un groupe L",
n et p valent entre 1 et 1000 et
L, L', L", P, P', Y et m ont la signification indiquée ci-dessus.

Une autre famille préférée de polyuréthanes associatifs cationiques est celle correspondant à la formule (IV) ci-dessus dans laquelle :
R et R' représentent tous les deux indépendamment un groupement hydrophobe, X, X' représentent chacun un groupe L", n et p valent 0, et L, L', L", Y et m ont la signification indiquée ci-dessus.

Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthanes associatifs cationiques est celle correspondant à la formule (Ia) ci-dessus dans laquelle :
R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthanes associatifs cationiques est comprise de préférence entre 400 et 500 000, en particulier entre 1000 et 400 000 et idéalement entre 1000 et 300 000.

Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel.

A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Lorsque X et/ou X' désignent un groupement comportant une amine tertiaire ou quaternaire, X et/ou X' peuvent représenter l'une des formules suivantes : 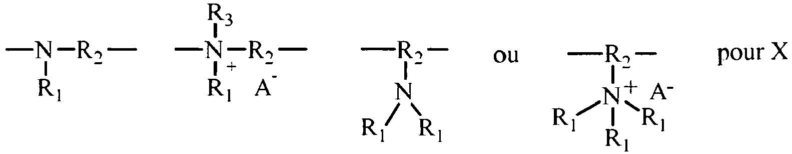 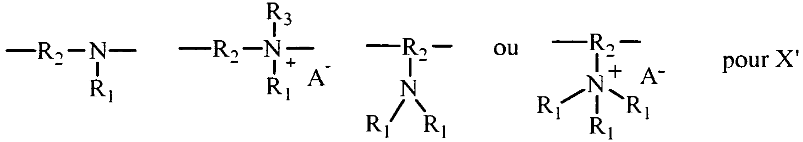 dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A- est un contre-ion physiologiquement acceptable.

Les groupements L, L' et L" représentent un groupe de formule : 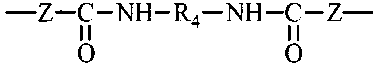 dans laquelle :
Z représente -O-, -S- ou -NH- ; et
R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 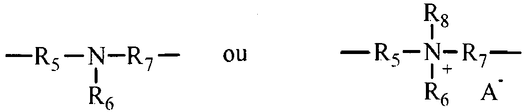 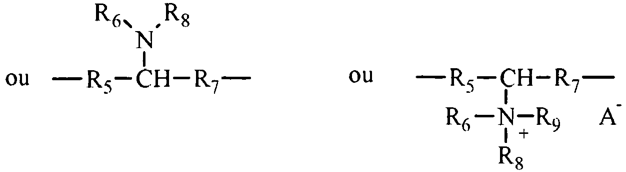 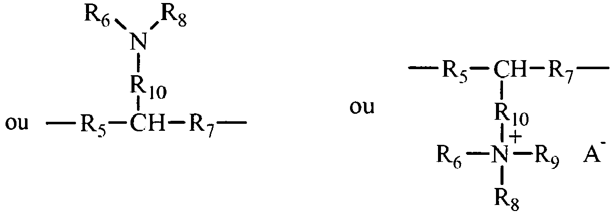 dans lesquelles :
R₅ et R₇ ont les mêmes significations que R₂ défini précédemment;
R₆, R₈ et R₉ ont les mêmes significations que R₁ et R₃ définis précédemment ;
R₁₀ représente un groupe alkylène, linéaire ou ramifié, éventuellement insaturé et pouvant contenir un ou plusieurs hétéroatomes choisis parmi N, O, S et P,
et A- est un contre-ion physiologiquement acceptable.

En ce qui concerne la signification de Y, on entend par groupement hydrophile, un groupement hydrosoluble polymérique ou non.

A titre d'exemple, on peut citer, lorsqu'il ne s'agit pas de polymères, l'éthylèneglycol, le diéthylèneglycol et le propylèneglycol.

Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs cationiques de formule (IV) utilisables selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" utilisable selon la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (IV) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.

Comme indiqué précédemment, ce composé peut comporter plus d'un motif à fonction amine. Il s'agit alors d'un polymère portant une répétition du motif à fonction amine.

Ce type de composés peut être représenté par l'une des formules suivantes :

HZ-(P)n-ZH,

ou

HZ-(P')p-ZH

dans lesquelles Z, P, P', n et p sont tels que définis plus haut.

A titre d'exemple de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (IV) est un diisocyanate correspondant à la formule :

O=C=N-R₄-N=C=O

dans laquelle R₄ est défini plus haut.

A titre d'exemple, on peut citer le méthylènediphényl-diisocyanate, le méthylènecyclohexanediisocyanate, l'isophorone-diisocyanate, le toluènediisocyanate, le naphtalènediisocyanate, le butanediisocyanate, l'hexanediisocyanate.

Un troisième composé entrant dans la préparation du polyuréthane de formule (IV) est un composé hydrophobe destiné à former les groupes hydrophobes terminaux du polymère de formule (IV).

Ce composé est constitué d'un groupe hydrophobe et d'une fonction à hydrogène labile, par exemple une fonction hydroxyle, amine primaire ou secondaire, ou thiol.

A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné alpha-hydroxyle.

Le groupe hydrophobe du polyuréthane de formule (IV) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.

A titre d'exemple, on peut citer, lorsqu'il ne s'agit pas de polymères, l'éthylèneglycol, le diéthylèneglycol et le propylèneglycol.

Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (IV) est facultatif. En effet, les motifs à fonction amine quaternaire ou protonée peuvent suffire à apporter la solubilité ou l'hydrodispersibilité nécessaire pour ce type de polymère dans une solution aqueuse.

Bien que la présence d'un groupe Y hydrophile soit facultative, on préfère cependant des polyuréthanes associatifs cationiques comportant un tel groupe.
- (II) les dérivés de cellulose quaternisée et les polyacrylates à groupements latéraux aminés non cycliques.

Les dérivés de cellulose quaternisée sont en particulier,
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthyl-celluloses quaternisées à chaînes grasses en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C12) et CRODACEL QS® (alkyle en C₁₈) commercialisés par la société CRODA.
- (III) le ou les polymères cationiques obtenus par polymérisation d'un mélange de monomères comprenant un ou plusieurs monomères vinyliques substitués par un ou plusieurs groupes amino, un ou plusieurs monomères vinyliques non ioniques hydrophobes, et un ou plusieurs monomères vinyliques associatifs.

En particulier, parmi ces polymères cationiques, on peut notamment citer le composé commercialisé par la société NOVEON sous la dénomination AQUA CC et qui correspond à la dénomination INCI POLYACRYLATE-1 CROSSPOLYMER.

Le POLYACRYLATE-1 CROSSPOLYMER est le produit de la polymérisation d'un mélange de monomères comprenant :
- un méthacrylate de di(alkyl en C₁-C₄) amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé (20-25 moles de motif oxyde d'éthylène),
- un allyl éther de polyéthylèneglycol/polypropylèneglycol 30/5,
- un méthacrylate d'hydroxy(alkyle en C₂-C₆), et
- un diméthacrylate d'éthylèneglycol.

Les polymères associatifs amphotères sont choisis de préférence parmi ceux comportant au moins un motif cationique non cyclique. Plus particulièrement encore, on préfère ceux préparés à partir ou comprenant 1 à 20 moles% de monomère comportant une chaîne grasse, et de préférence 1,5 à 15 moles% et plus particulièrement encore 1,5 à 6 moles%, par rapport au nombre total de moles de monomères.

Les polymères associatifs amphotères préférés selon l'invention comprennent, ou sont préparés en copolymérisant au moins un monomère de formule (V) ou (VI) : 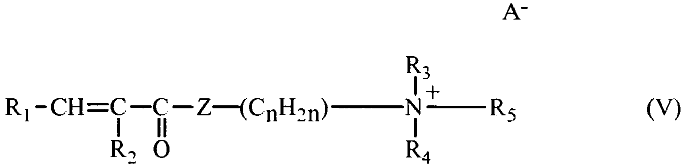 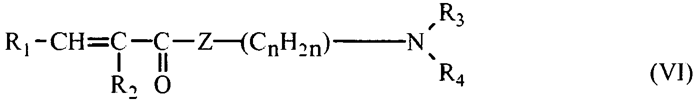
dans lesquelles, R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
Z représente un groupe NH ou un atome d'oxygène,
n est un nombre entier de 2 à 5,
A- est un anion issu d'un acide organique ou minéral, tel qu'un anion méthosulfate ou un halogénure tel que chlorure ou bromure;

2) au moins un monomère de formule (VII)

   R₆-CH=CR₇⁻COOH (VII)

   dans laquelle, R₆ et R₇, identiques ou différents, représentent un atome d'hydrogène ou un radical méthyle;
   et
3) au moins un monomère de formule (VIII) :

   R₆-CH=CR₇-COXR₈ (VIII)

   dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ;
   l'un au moins des monomères de formule (V), (VI) ou (VII) comportant au moins une chaîne grasse.

Les monomères de formule (V) et (VI) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,
ces monomères étant éventuellement quaternisés, par exemple par un halogénure d'alkyle en C₁-C₄ ou un sulfate de dialkyle en C₁-C₄.

Plus particulièrement, le monomère de formule (V) est choisi parmi le chlorure d'acrylamidopropyl triméthyl ammonium et le chlorure de méthacrylamidopropyl triméthyl ammonium.

Les monomères de formule (VII) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (VII) est l'acide acrylique.

Les monomères de formule (VIII) de la présente invention sont choisis, de préférence, dans le groupe constitué par des acrylates ou méthacrylates d'alkyle en C₁₂-C₂₂ et plus particulièrement en C₁₆-C₁₈-Les monomères constituant les polymères amphotères à chaîne grasse de l'invention sont de préférence déjà neutralisés et/ou quaternisés.

Le rapport du nombre de charges cationiques/charges anioniques est de préférence égal à environ 1.

Les polymères associatifs amphotères selon l'invention comprennent de préférence de 1 à 10 % moles du monomère comportant une chaîne grasse (monomère de formule (V), (VI) ou (VIII), et de préférence de 1,5 à 6% moles.

Les poids moléculaires moyens en poids des polymères associatifs amphotères selon l'invention peuvent varier de 500 à 50.000.000 et sont de préférence compris entre 10.000 et 5 000 000.

Les polymères associatifs amphotères selon l'invention peuvent également contenir d'autres monomères tels que des monomères non ioniques et en particulier tels que les acrylates ou méthacrylates d'alkyle en C₁-C₄.

Des polymères associatifs amphotères selon l'invention sont par exemple décrits et préparés dans la demande de brevet WO9844012.

Parmi les polymères associatifs amphotères selon l'invention, on préfère les terpolymères acide acrylique/chlorure de (méth)acrylamidopropyl triméthyl ammonium/ méthacrylate de stéaryle.

Les polymères associatifs de type non ionique utilisables selon l'invention sont choisis de préférence parmi :
- (1) les celluloses modifiées par des groupements comportant au moins une chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
   - celles modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL.
- (2) les hydroxypropylguars modifiés par des groupements comportant au moins une chaîne grasse tel que le produit ESAFLOR HM 22® (chaîne alkyle en C₂₂) vendu par la société LAMBERTI, les produits RE210-18® (chaîne alkyle en C₁₄) et RE205-1® (chaîne alkyle en C₂₀) vendus par la société RHONE POULENC.
- (3) les copolymères de vinyl pyrrolidone et de monomères hydrophobes à chaîne grasse dont on peut citer à titre d'exemple :
   - les produits ANTARON V216® ou GANEX V216® (copolymère vinylpyrrolidone / hexadécène) vendu par la société I.S.P.
   - les produits ANTARON V220® ou GANEX V220® (copolymère vinylpyrrolidone / eicosène) vendu par la société I.S.P.
- (4) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208®.
- (5) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- (6) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
- (7) les polymères à squelette aminoplaste éther possédant au moins une chaîne grasse, tels que les composés PURE THIX® proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut aussi utiliser aussi le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C₁₂-₁₄ et le produit ELFACOS T212® à chaîne alkyle en C₁₈ de chez AKZO.

Le produit DW 1206B® de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

De préférence, les polymères épaississants selon l'invention sont choisis parmi les composés de la famille (II), en particulier les terpolymères acide acrylique/méthacrylate de lauryle / vinylpyrrolidone.

Le ou les polymères épaississants est ou sont présents dans la composition selon l'invention dans une teneur allant de 0,01 à 20 % en poids et de préférence de 0,5 à 5 % en poids par rapport au poids total de la composition, mieux de 1 à 4 % en poids par rapport au poids total de la composition.

De préférence, le rapport pondéral entre la quantité totale de copolymère(s) vinylformamide / vinylformamine d'une part et la quantité totale de polymères épaississants d'autre part, est compris entre 0,1 et 20, de préférence entre 1 et 15, et mieux entre 1 et 8.

Les compositions selon l'invention peuvent également contenir un ou plusieurs corps gras.

Par corps gras, on entend au sens de la présente invention un composé organique qui, à température ambiante (25°C) et à pression atmosphérique, est insoluble dans l'eau (c'est-à-dire, présente une solubilité dans l'eau inférieure à 1% en poids et de préférence inférieure à 0,5% en poids), et est soluble dans au moins un solvant organique (par exemple l'éthanol, le chloroforme, ou le benzène) à au moins 1% en poids.

Les corps gras non siliconés utilisables dans les compositions selon la présente invention sont notamment toutes les huiles, cires, résines non siliconées organiques ou minérales, naturelles ou synthétiques répondant à cette définition.

Une huile, au sens de la présente invention, est un composé lipophile, liquide à température ambiante, à changement d'état solide/liquide réversible.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arana, de tournesol, de ricin, d'avocat, les triglycérides des acides caprylique/caprique comme par exemple ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R⁶COOR⁷ et R⁶OR⁷ dans laquelle R⁶ représente une chaîne hydrocarbonée saturée ou insaturée (par exemple, le reste d'un acide gras) comportant de 8 à 29 atomes de carbone, et R⁷ représente une chaîne hydrocarbonée, ramifiée ou non, contenant de 3 à 30 atomes de carbone ; citons par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononaoate de diéthylèneglycol ; et les esters de pendaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, l'huile de vaseline, les polydécènes, le polyisobutène hydrogéné tel que l'huile de parléam ;
- les alcools gras fluides ayant de 8 à 26 atomes de carbone, comme par exemple l'octyldodécanol, le 2-butyloctanol, l'alcool oléique, l'alcool linoléique ou l'alcool linolénique ;
- les huiles fluorées partiellement hydrocarbonées comme celles décrites dans le document JP-A-2 295912. Comme huiles
fluorées, on peut citer aussi le perfluorométhylcyclopentane et le perfluoro-1,3- diméthylcyclohexane, par exemple vendus sous les dénominations de « FLUTEC PC1® » et « FLUTEC PC3® » par la Société BNFL Fluorochemicals ; le perfluoro-1,2-diméthylcyclobutane ; les perfluoralcanes tels que le dodécafluoropentane et le tétradécafluorohexane, par exemple vendus sous les dénominations de « PF 5050® » et « PF 5060® » par la Société 3M, ou encore le bromoperfluorooctyle par exemple vendu sous la dénomination « FORALKYL® » par la Société Atochem ; le nanofluorométhoxybutane par exemple vendu sous la dénomination « MSX 4518® » par la Société 3M et le nanofluoroéthoxyisobutane ; les dérivés de perfluoromorpholine, tels que la 4-trifluorométhyl perfluoromorpholine par exemple vendue sous la dénomination « PF 5052® » par la Société 3M.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Une cire, au sens de la présente invention, est un composé lipophile, solide à température ambiante (environ 25 °C), à changement d'état solide/liquide réversible, ayant une température de fusion supérieure à environ 40 °C et pouvant aller jusqu'à 200 °C, et présentant à l'état solide une organisation cristalline anisotrope. Les cires animales et végétales comprennent comme constituants essentiels des esters d'acides carboxyliques et d'alcools à longues chaînes. D'une manière générale, la taille des cristaux de la cire est telle que les cristaux diffractent et/ou diffusent la lumière, conférant à la composition qui les comprend un aspect trouble plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange, détectable microscopiquement et macroscopiquement (opalescence).

A titre de cires utilisables dans la présente invention, on peut citer les cires d'origine animale telles que la cire d'abeille, le spermaceti, la cire de lanoline et les dérivés de lanoline ; les cires végétales telles que les cires de tournesol, de riz, de pomme, la cire de Carnauba, de Candellila, d'Ouricury, du Japon, le beurre de cacao ou les cires de fibres de liège ou de canne à sucre ; les cires minérales, par exemple, de paraffine, de vaseline, de lignite ou les cires microcristallines, la cérésine ou l'ozokérite ; les cires synthétiques telles que les cires de polyéthylènes, les cires de Fischer-Tropsch ; les esters d'acides gras cireux ; les alcools gras cireux tels que par exemple les alcools myristylique, cétylique, stéarylique, arachidylique, béhénylique et érucylique , et leurs mélanges.

Comme huile végétale, on peut notamment mentionner l'huile d'amande douce, l'huile d'avocat, l'huile de ricin, l'huile d'olive, la cire liquide de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, l'huile de palme, l'huile de noyau d'abricot, l'huile de calophyllum, l'huile d'onagre, le beurre de karité, l'huile de son de riz, l'huile de germes de maïs, l'huile de passiflore et l'huile de seigle.

Comme huile animale, on peut notamment citer le perhydrosqualène.

Comme huile minérale, on peut notamment citer l'huile de paraffine et l'huile de vaseline.

Comme huile synthétique, on peut notamment citer le squalane, les poly(α-oléfines) comme l'isododécane ou l'isohexadécane, les huiles végétales transestérifiées, les huiles fluorées, les esters gras.

Par esters gras, on désigne les composés de formule RₐCOOR_{b} dans laquelle Rₐ représente le reste d'un acide supérieur linéaire ou ramifié, hydroxylé ou non, saturé ou non, comportant de 4 à 29 atomes de carbone et R_{b} représente une chaîne hydrocarbonée linéaire ou ramifiée, saturée ou non contenant de 3 à 30 atomes de carbone, le nombre total d'atomes de carbone de l'ester étant supérieur à 10. A titre d'exemples non limitatifs, on peut notamment citer l'huile de purcellin (octanoate de stéaryle), le myristate d'isopropyle, le palmitate d'isopropyle, le stéarate de butyle, le laurate d'hexyle, l'isononanoate d'isononyle, le palmitate de 2-éthylhexyle, le laurate de 2-hexyldécyle, le palmitate de 2-octyldécyle, le myristate de 2-octyldodécyle, le néopentanoate d'isostéaryle ou le néopentanoate de tridécyle.

Les huiles fluorées peuvent être partiellement hydrocarbonées et/ou siliconées, comme par exemple celles décrites dans le document JP-A-2-295912.

Les alcools gras préférés comprennent entre autres les alcools myristylique, cétylique, stéarylique, arachidylique, béhénylique et érucylique.

Une cire, au sens de la présente invention, est un composé lipophile, solide à température ambiante (environ 25 °C), à changement d'état solide/liquide réversible, ayant une température de fusion supérieure à environ 40°C et pouvant aller jusqu'à 200°C, et présentant à l'état solide une organisation cristalline anisotrope. Les cires animales et végétales comprennent comme constituants essentiels des esters d'acides carboxyliques et d'alcools à longues chaînes. D'une manière générale, la taille des cristaux de la cire est telle que les cristaux diffractent et/ou diffusent la lumière, conférant à la composition qui les comprend un aspect trouble plus ou moins opaque. En portant la cire à sa température de fusion, il est possible de la rendre miscible aux huiles et de former un mélange homogène microscopiquement.

A titre de cires utilisables dans la présente invention, on peut citer les cires d'origine animale telles que la cire d'abeille, le spermaceti, la cire de lanoline et les dérivés de lanoline ; les cires végétales telles que les cires de tournesol, de riz, de pomme, la cire de Carnauba, de Candellila, d'Ouricury, du Japon, le beurre de cacao ou les cires de fibres de liège ou de canne à sucre ; les cires minérales, par exemple, de paraffine, de vaseline, de lignite ou les cires microcristallines, la cérésine ou l'ozokérite ; les cires synthétiques telles que les cires de polyéthylènes, les cires de Fischer-Tropsch, et leurs mélanges.

### Tensioactifs

Selon un mode de réalisation préféré, la composition selon l'invention contient moins de 5% en poids au total de tensioactifs anioniques et de tensioactifs non ioniques, de préférence moins de 3% en poids, et de manière encore plus préférée moins de 1 % en poids, mieux moins de 0,5 % en poids par rapport au poids total de la composition.

Encore plus préférentiellement, les tensioactifs anioniques et les tensioactifs non ioniques sont totalement absents des compositions selon l'invention.

La composition selon l'invention peut contenir un ou plusieurs tensioactifs anioniques et/ou non ioniques, du moment que la teneur totale en tensioactifs anioniques et en tensioactifs non ioniques reste, de préférence, inférieure aux valeurs indiquées ci-avant.

A titre d'exemple de tensioactifs anioniques susceptibles d'être présents dans les compositions selon la présente invention, on peut citer notamment (liste non limitative) les sels (en particulier les sels alcalins, notamment de sodium, les sels d'ammonium, les sels d'amines, les sels d'aminoalcools ou les sels d'alcalinoterreux (de magnésium) des composés suivants : les alkylsulfates, les alkyléthersulfates, alkylamidoéthersulfates, alkylarylpolyéthersulfates, les monoglycérides sulfates ; les alkylsulfonates, les alkylphosphates, les alkylamidesulfonates, alkylarylsulfonates, les α-oléfine-sulfonates, lesparaffine-sulfonates ; les alkylsulfosuccinates ; les alkyléthersulfosuccinates, les alkylamidesulfosuccinates ; les alkylsulfosuccinamates ; les alkylsulfoacétates ; les alkylétherphosphates, les acylsarcosinates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle.

Parmi les tensioactifs anioniques, on peut également citer les sels d'acides gras tels que les sels acides oléique, ricinoléique, palmitique, stéarique ; les acides d'huile de coprah ou d'huile de coprah hydrogénée ; les acyl-lactylates dont le radical acyle comporte 8 à 20 atomes de carbone.

On peut également citer les tensioactifs faiblement anioniques, comme les acides d'alkyl D-galactoside uroniques et leurs sels ainsi que les acides alkyl (C₆ - C₂₄) éther carboxyliques polyoxyalkylénés, les acides alkyl (C₆-C₂₄)aryl éther carboxyliques polyoxyalkylénés, les acides alkyl (C₆-C₂₄) amino éther carboxyliques polyoxyalkylénés et les sels de ces acides, en particulier ceux comportant de 2 à 50 groupements oxyde d'éthylène et leurs mélanges.

Les tensioactifs non-ioniques susceptibles d'être présents dans les compositions de la présente invention sont des composés bien connus en soi (voir notamment à cet égard "Handbook of Surfactants" par M.R. PORTER, éditions Blackie & Son (Glasgow and London), 1991, pp 116-178). Ils sont choisis notamment parmi les alcools et alcools gras polyéthoxylés, polypropoxylés ou polyglycérolés, les alpha-diols polyéthoxylés, polypropoxylés ou polyglycérolés, les alkyl(C₁-₂₀)phénols polyéthoxylés, polypropoxylés ou polyglycérolés ou les acides gras polyéthoxylés, polypropoxylés ou polyglycérolés, la chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras et de sorbitane éthoxylés ayant de 2 à 30 motifs d'oxyde d'éthylène, les esters d'acides gras du saccharose, les esters d'acides gras et de polyéthylèneglycol, les alkylpolyglycosides, les huiles végétales polyéthoxylées, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

La composition selon l'invention peut par ailleurs contenir un ou plusieurs tensioactifs amphotères ou zwittérioniques.

Les tensioactifs amphotères ou zwittérioniques utilisables dans les compositions de la présente invention comprennent par exemple les dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer également les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3ème édition, 1982, sous les dénominations Amphocarboxy-glycinate et Amphocarboxypropionate de structures respectives (1) et (2) :

Rₐ-CONHCH₂CH₂-N(R_{b})(R_{c})(CH₂COO⁻) (1)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

Rₐ'-CONHCH₂CH₂-N(B)(B') (2)

dans laquelle :
B représente -CH₂CH₂OX',
B' représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

La composition selon l'invention peut comprendre de 0,01 à 10 % en poids de tensioactif(s) amphotère(s) ou zwittérionique(s), et de manière plus préférée de 0,05 à 4 % en poids, par rapport au poids total de la composition.

Selon un mode de réalisation particulièrement préféré de la présente invention, la composition selon l'invention est une composition non détergente, c'est-à-dire qu'elle contient moins de 5% en poids au total de tensioactifs détergents, de préférence moins de 3% en poids, de manière plus préférée moins de 1% en poids, et de manière encore plus préférée moins de 0,5% en poids, par rapport au poids total de la composition. Par tensioactifs détergents, on désigne selon ce mode de réalisation les tensioactifs anioniques, non ioniques, amphotères et zwittérioniques.

La composition selon l'invention peut par ailleurs contenir un ou plusieurs tensioactifs cationiques.

Les tensioactifs cationiques utilisables dans les compositions de la présente invention comprennent par exemple les sels d'amines grasses primaires, secondaires ou tertiaires, éventuellement polyoxyalkylénées, les sels d'ammonium quaternaire, et leurs mélanges.

A titre de sels d'ammonium quaternaire, on peut notamment citer, par exemple :
- ceux répondant à la formule générale (IX) suivante : 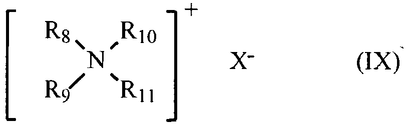 dans laquelle les radicaux R₈ à R₁₁, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle en C₁₋₃₀, alcoxy en C₁₋₃₀, polyoxyalkylène (C₂-C₆), alkylamide en C₁₋₃₀, alkyl(C₁₂-C₂₂)amidoalkyle(C₂-C₆), alkyl(C₁₂-C₂₂)acétate, et hydroxyalkyle en C₁₋₃₀ ; X est un anion choisi dans le groupe des halogénures, phosphates, acétates, lactates, alkyl(C₂-C₆)sulfates, alkyl- ou alkylaryl-sulfonates.

Parmi les sels d'ammonium quaternaire de formule (I), on préfère d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL^{®} 70 par la société VAN DYK.
- les sels d'ammonium quaternaire de l'imidazoline, comme par exemple ceux de formule (X) suivante : 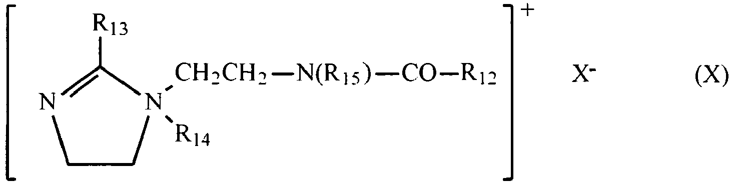 dans laquelle R₁₂ représente un radical alcényle ou alkyle comportant de 8 à 30 atomes de carbone, par exemple dérivés des acides gras du suif, R₁₃ représente un atome d'hydrogène, un radical alkyle en C₁-C₄ ou un radical alcényle ou alkyle comportant de 8 à 30 atomes de carbone, R₁₄ représente un radical alkyle en C₁-C₄, R₁₅ représente un atome d'hydrogène, un radical alkyle en C₁-C₄, X⁻ est un anion choisi dans le groupe des halogénures, phosphates, acétates, lactates, alkylsulfates, alkyl- ou alkylaryl-sulfonates. De préférence, R₁₂ et R₁₃ désignent un mélange de radicaux alcényle ou alkyle comportant de 12 à 21 atomes de carbone, par exemple dérivés des acides gras du suif, R₁₄ désigne un radical méthyle, R₁₅ désigne un atome d'hydrogène. Un tel produit est par exemple commercialisé sous la dénomination REWOQUAT^{®} W 75 par la société REWO ;
- les sels de diammonium quaternaire de formule (XI) suivante: 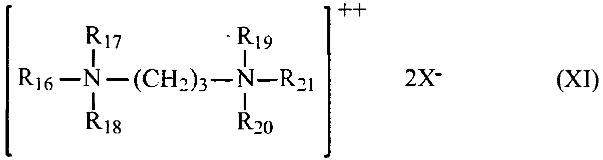 dans laquelle R₁₆ désigne un radical aliphatique comportant environ de 16 à 30 atomes de carbone ; R₁₇, R₁₈, R₁₉, R₂₀ et R₂₁, identiques ou différents sont choisis parmi un atome d'hydrogène et un radical alkyle comportant de 1 à 4 atomes de carbone ; et X est un anion choisi dans le groupe des halogénures, acétates, phosphates, nitrates et méthylsulfates. De tels sels de diammonium quaternaire comprennent notamment le dichlorure de propanesuif diammonium ;
- les sels d'ammonium quaternaire contenant au moins une fonction ester, tels que ceux de formule (XII) suivante : 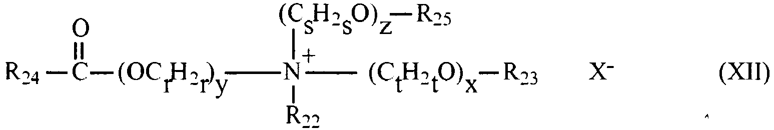 dans laquelle :
   R₂₂ est choisi parmi les radicaux alkyles en C₁-C₆ et les radicaux hydroxyalkyles ou dihydroxyalkyles en C₁-C₆ ,
   R₂₃ est choisi parmi :
      - le radical 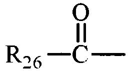
      - les radicaux R₂₇ hydrocarbonés en C₁-C₂₂, linéaires ou ramifiés, saturés ou insaturés,
      - l'atome d'hydrogène,
   R₂₅ est choisi parmi :
      - le radical 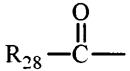
      - les radicaux R₂₉ hydrocarbonés en C₁-C₆, linéaires ou ramifiés, saturés ou insaturés,
      - l'atome d'hydrogène,
   R₂₄, R₂₆ et R₂₈, identiques ou différents, sont choisis parmi les radicaux hydrocarbonés en C₇-C₂₁, linéaires ou ramifiés, saturés ou insaturés ;
   r, s et t, identiques ou différents, sont des entiers valant de 2 à 6 ;
   y est un entier valant de 1 à 10 ;
   x et z, identiques ou différents, sont des entiers valant de 0 à 10 ;
   X- est un anion simple ou complexe, organique ou inorganique ;
   sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (XII) dans laquelle :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
   - le radical 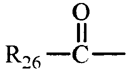
   - les radicaux méthyle, éthyle ou hydrocarbonés en C₁₄-C₂₂,
   - l'atome d'hydrogène ;
- R₂₅ est choisi parmi :
   - le radical 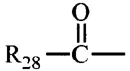
   - l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (XII) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthyl ammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART^{®} par la société HENKEL, STEPANQUAT^{®} par la société STEPAN, NOXAMIUM^{®} par la société CECA, REWOQUAT^{®} WE 18 par la société REWO-WITCO.

La composition selon l'invention peut contenir par exemple un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

La composition selon l'invention peut comprendre de 0,01 à 10 % en poids de tensioactif(s) cationique(s), et de manière plus préférée de 0,05 à 4 % en poids, par rapport au poids total de la composition.

Les compositions selon l'invention peuvent également comprendre en outre une ou plusieurs silicone sous forme soluble, dispersée ou micro-dispersée. Les silicones sont alors de préférence présents en une quantité allant de 0,01 à 10% en poids, et plus préférentiellement de 0,1 à 5% en poids, par rapport au poids total de la composition.

A titre d'exemple, on peut notamment citer les huiles siliconées, telles que par exemple les polydiméthylsiloxanes linéaires ou cycliques.

Les compositions conformes à l'invention peuvent être conditionnées par exemple dans un pot, dans un tube, un flacon pompe, ou dans un dispositif aérosol usuel en cosmétique.

Les compositions selon l'invention peuvent, lorsqu'elles ont destinées à être conditionnées dans un dispositif de type aérosol, contenir un ou plusieurs gaz propulseurs.

Le gaz propulseur peut alors être choisi par exemple parmi le diméthyléther, les alcanes en C₃ à C₅, les hydrocarbures halogénés, et leurs mélanges.

Les compositions selon l'invention peuvent en outre contenir un ou plusieurs additifs choisis parmi les agents nacrants ; les agents opacifiants ; les agents plastifiants ; les filtres solaires ; les parfums ; les colorants ; les conservateurs ; les agents de stabilisation du pH ; les acides ; les bases ; les polyols (par exemple les glycols) ; les charges minérales ; les paillettes, et tout autre additif classiquement utilisé dans le domaine cosmétique.

L'homme de métier veillera à choisir les éventuels additifs et leurs quantités de manière à ce qu'ils ne nuisent pas aux propriétés des compositions de la présente invention.

Ces additifs peuvent être présents dans la composition selon l'invention en une quantité allant de 0 à 50 % en poids par rapport au poids total de la composition.

Les compositions selon l'invention peuvent se présenter entre autres sous forme de liquides plus ou moins épaissis, de gels, de crèmes, de pâtes ou de mousses

De préférence, elles se présentent sous forme de gels.

La composition selon l'invention peut également se présenter sous la forme d'une composition en deux parties, destinées à être mélangées au moment de l'emploi.

La présente invention a donc également pour objet une composition cosmétique en deux parties, comprenant :
- une première partie comprenant, dans un milieu cosmétiquement acceptable, un ou plusieurs copolymères vinylformamide / vinylformamine tel que décrit ci-avant, lequel comprend :
   de 20 à 40 % en moles de motifs de formule suivante A : 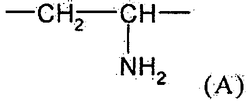 et de 60 à 80 % en moles de motifs de formule suivante B: 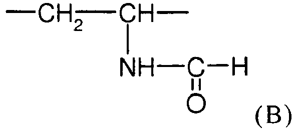 tels que définis précédemment et
   - une seconde partie comprenant, dans un milieu cosmétiquement acceptable, un ou plusieurs polymères épaississants associatifs différents des copolymères vinylformamide / vinylformamine.

Avantageusement, la composition résultant du mélange desdites première et seconde parties comprenant moins de 5% en poids au total de tensioactifs anioniques et de tensioactifs non ioniques.

La description faite ci-avant des divers ingrédients de la composition en une partie selon l'invention concerne s'applique également à la composition en deux parties, lesdits ingrédients pouvant être présents dans l'une ou l'autre partie à l'exception du ou des copolymères vinylformamide / vinylformamine et du ou des polymères épaississants, qui sont conditionnés séparément. De même, la description ci-avant des teneurs et des rapports pondéraux des divers ingrédients, y compris le ou les copolymères vinylformamide / vinylformamine et le ou les polymères épaississants s'applique également à la composition en deux parties, étant entendu que ces teneurs et rapports pondéraux s'appliquent à la composition finale obtenue après mélange des deux parties.

Par ailleurs la composition en deux parties selon l'invention peut être conditionnées dans un dispositif à plusieurs compartiments ou « kit ».

La présente invention concerne donc également un dispositif comprenant au moins deux compartiments, dont un premier compartiment renferme une première composition comprenant, dans un milieu cosmétiquement acceptable, un ou plusieurs copolymères vinylformamide / vinylformamine tels que décrits ci-avant, et un second compartiment renferme une seconde composition comprenant, dans un milieu cosmétiquement acceptable, un ou des polymères épaississants associatifs tels que décrits ci-avant.

La composition selon l'invention peut être avantageusement utilisée pour le traitement cosmétique des cheveux. En particulier, elle peut être employée pour le coiffage des cheveux, par exemple pour la mise en forme et/ou la fixation de la coiffure.

Selon un mode de réalisation particulièrement préféré, elle est utilisée pour le coiffage et le conditionnement simultanés des cheveux.

La présente invention concerne également un procédé de traitement cosmétique des cheveux, par exemple un procédé de soin capillaire, ou un procédé de mise en forme et/ou de maintien de la coiffure, qui consiste à appliquer sur les cheveux une quantité efficace d'une composition telle que décrite ci-dessus, puis à effectuer un éventuel rinçage après un éventuel temps de pose.

De préférence, la composition selon l'invention n'est pas rincée.

Les exemples suivants sont donnés à titre illustratif de la présente invention. Dans ces exemples, toutes les quantités sont indiquées en pour cent en poids de matière active (MA) par rapport au poids total de la composition.

### Exemples

Ces exemples illustrent la formulation de gels de coiffage.

Un premier gel a été préparé à partir des ingrédients indiqués dans le tableau ci-dessous (hors invention):

| | | | | |
|---|---|---|---|---|
| Polyvinylformamide hydrolysé à 30% | | (1) | | 5% |
| | | | | |
| Gomme de guar | (2) | | | 2% |
| | | | | |
| CONSERVATEUR | | | | 0.3% |
| | | | | |
| EAU | | QSP 100% | | |

Un deuxième gel a été préparé à partir des ingrédients indiqués dans le tableau ci-dessous :

| | |
|---|---|
| Polyvinylformamide hydrolysé à 30% (1) | 6% |
| | |
| TERPOLYMERE ACIDE ACRYLIQUE / VINYLPYRROLIDONE / METHACRYLATE DE LAURYLE | |
| ACRYLIDONE LM (ISP) | 2 % |
| | |
| Triéthanolamine | qs pH = 8 |
| | |
| EAU | QSP 100% |

(1) commercialisé sous la dénomination LUPAMIN 9030 par la société BASF.
(2) commercialisé sous la dénomination JAGUAR HP 105 par la société RHODIA.

### Résultats obtenus :

Les performances des deux compositions décrites ci-avant ont été évaluées par des professionnels, sur des panels de modèles.

Ces compositions ont permis d'obtenir une très bonne fixation des cheveux, avec une excellente tenue dans le temps et une bonne résistance aux sollicitations mécaniques.

En outre, ces compositions se sont avérées apporter aux cheveux d'excellentes propriétés cosmétiques, en particulier en thermes de douceur.

On a réalisé les deux compositions suivantes (hors invention):

| | Composition1 | Composition2 |
|---|---|---|
| Polyvinylformamide hydrolysée à 30% (1) | 5%ma | - |
| Poly N-vinylformamide (2) | - | 5%ma |
| Gomme de guar (3) | 2%ma | 2%ma |
| Eau déminéralisée | Qsp100 | Qsp100 |

(1) Commercialisé sous la dénomination LUPAMIN 9030 par la société BASF
(2) commercialisé sous la dénomination LUPAMIN 9000 par la société BASF
(3) commercialisé sous la dénomination JAGUAR HP 105 par la société RHODIA

Les deux compositions précédentes ont été appliquées, à raison de 1g de formulation par mèche sur mèches de cheveux châtains humides de 2.7g préalablement shampouinées, rincées et essorées.

En final les mèches, non rincées, ont ensuite été séchées sous casque pendant 30 minutes, puis démêlées.

Un panel de 7 testeurs a évalué le caractère lisse des cheveux traités avec les compositions 1 ou 2 en les classant suivant ce critère : rang 1 pour la mèche avec les cheveux présentant le toucher le plus lisse, rang 2 pour la mèche avec les cheveux présentant le toucher le moins lisse

Les 7 évaluateurs ont trouvé un toucher plus lisse avec la composition 1. Somme de rangs :7 pour la composition 1 , 14 pour la composition 2

Selon le test de KRAMER (A non parametric ranking method for the statistical evaluation of sensory data, chemical Senses and Flavor (1974)121-123), au seuil de 5%,7 étant inférieur à l'intervale 8-13, le toucher est significativement plus lisse avec la composition 1.

## Revendications

1. Composition cosmétique comprenant, dans un milieu cosmétiquement acceptable :
- un ou plusieurs copolymères vinylformamide / vinylformamine comprenant :
de 20 à 40 % en moles de motifs de formule suivante A : 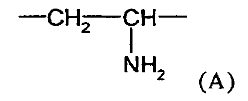 et de 60 à 80 % en moles de motifs de formule suivante B: 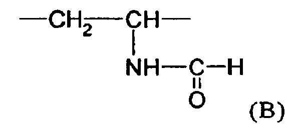 le ou les copolymères vinylformamide / vinylformamine étant constitués uniquement de motifs de formule A et de motifs de formule B,
et
- un ou plusieurs polymères épaississants associatifs, différents des copolymères vinylformamide / vinylformamine.

2. Composition selon la revendication précédente, **caractérisée en ce que** le ou les copolymères vinylformamide / vinylformamine sont présents dans des proportions allant de 0,1 à 25 % en poids, par rapport au poids total de la composition.

3. Composition selon l'une quelconque des revendications précédentes, **caractérisée en ce que** les épaississants sont présents dans les compositions dans des proportions allant, de préférence, de 0,01 à 20 % en poids, plus préférentiellement de 0,05 à 5 % en poids et plus particulièrement de 1 à 4 % en poids, par rapport au poids total de la composition.

4. Composition selon l'une quelconque des revendications précédentes, **caractérisée en ce que** le rapport pondéral entre la quantité totale de copolymère(s) vinylformamide / vinylformamine d'une part et la quantité totale d'épaississant d'autre part, est compris entre 0,1 et 20, de préférence entre 1 et 15, et mieux entre 1 et 8.

5. Composition selon l'une quelconque des revendications précédentes, **caractérisée en ce qu'**elle comprend également de 0,01 à 10 % en poids de tensioactif(s) amphotère(s) ou zwittérionique(s), par rapport au poids total de la composition.

6. Composition selon l'une quelconque des revendications précédentes, **caractérisée en ce qu'**elle comprend également de 0,01 à 10 % en poids de tensioactif(s) cationique(s), par rapport au poids total de la composition.

7. Composition selon l'une quelconque des revendications précédentes, **caractérisée en ce qu'**elle contient en outre un ou plusieurs additifs choisis parmi les agents nacrants ; les agents opacifiants ; les agents plastifiants ; les filtres solaires ; les parfums ; les colorants ; les conservateurs ; les agents de stabilisation du pH ; les acides ; les bases ; les polyols; les charges minérales ; les paillettes, et tout autre additif classiquement utilisé dans le domaine cosmétique.

8. Composition cosmétique en deux parties, comprenant :
- une première partie comprenant, dans un milieu cosmétiquement acceptable, un ou plusieurs copolymères vinylformamide / vinylformamine, lequel comprend :
de 20 à 40 % en moles de motifs de formule suivante A : 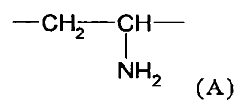 et de 60 à 80 % en moles de motifs de formule suivante B: 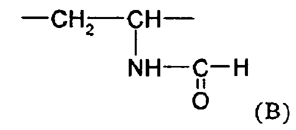 le ou les copolymères vinylformamide / vinylformamine étant constitués uniquement de motifs de formule A et de motifs de formule B,
et
- une seconde partie comprenant, dans un milieu cosmétiquement acceptable, un ou plusieurs épaississants associatifs, différents des copolymères vinylformamide / vinylformamine.

9. Procédé de traitement cosmétique des cheveux, **caractérisé en ce qu'**il consiste à appliquer sur les cheveux une quantité efficace d'une composition selon l'une quelconque des revendications 1 à 8, puis à effectuer un éventuel rinçage après un éventuel temps de pose.

10. Utilisation d'une composition selon l'une quelconque des revendications 1 à 8, pour la mise en forme et/ou la fixation de la coiffure.

## Patentansprüche

1. Kosmetische Zusammensetzung, die in einem kosmetisch unbedenklichen Medium:
- ein oder mehrere Vinylformamid/Vinylformamin-Copolymere, umfassend:
20 bis 40 Mol-% Einheiten der folgenden Formel A: 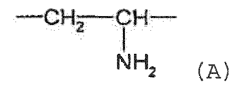 und 60 bis 80 Mol-% Einheiten der folgenden Formel B: 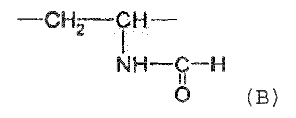 wobei das bzw. die Vinylformamid/Vinylformamin-Copolymere ausschließlich aus Einheiten der Formel A und Einheiten der Formel B aufgebaut ist bzw. sind,
und
- ein oder mehrere Assoziativverdicker-Polymere, die von den Vinylformamid/Vinylformamin-Copolymeren verschieden sind,
umfasst.

2. Zusammensetzung nach dem vorhergehenden Anspruch, **dadurch gekennzeichnet, dass** das bzw. die Vinylformamid/Vinylformamin-Copolymere in Anteilen im Bereich von 0,1 bis 25 Gew.-%, bezogen auf das Gesamtgewicht der Zusammensetzung, vorliegen.

3. Zusammensetzung nach einem der vorhergehenden Ansprüche, **dadurch gekennzeichnet, dass** die Verdicker in den Zusammensetzungen in Anteilen im Bereich von vorzugsweise 0,01 bis 20 Gew.-%, weiter bevorzugt 0,05 bis 5 Gew.-% und spezieller 1 bis 4 Gew.-%, bezogen auf das Gesamtgewicht der Zusammensetzung, vorliegen.

4. Zusammensetzung nach einem der vorhergehenden Ansprüche, **dadurch gekennzeichnet, dass** das Gewichtsverhältnis zwischen der Gesamtmenge an Vinylformamid/Vinylformamin-Copolymer(en) einerseits und der Gesamtmenge an Verdicker andererseits zwischen 0,1 und 20, vorzugsweise zwischen 1 und 15 und noch besser zwischen 1 und 8 liegt.

5. Zusammensetzung nach einem der vorhergehenden Ansprüche, **dadurch gekennzeichnet, dass** sie außerdem 0,01 bis 10 Gew.-% eines oder mehrerer amphoterer oder zwitterionischer Tenside, bezogen auf das Gesamtgewicht der Zusammensetzung, umfasst.

6. Zusammensetzung nach einem der vorhergehenden Ansprüche, **dadurch gekennzeichnet, dass** sie außerdem 0,01 bis 10 Gew.-% eines oder mehrerer kationischer Tenside, bezogen auf das Gesamtgewicht der Zusammensetzung, umfasst.

7. Zusammensetzung nach einem der vorhergehenden Ansprüche, **dadurch gekennzeichnet, dass** sie des Weiteren ein oder mehrere Additive enthält, die aus Perlglanzmitteln; Trübungsmitteln; Weichmachern; Lichtschutzmitteln; Duftstoffen; Farbmitteln; Konservierungsstoffen; Mitteln zur Stabilisierung des pH-Werts; Säuren; Basen; Polyolen; mineralischen Füllstoffen; Pailletten und allen anderen Additiven, die herkömmlicherweise auf dem Gebiet der Kosmetik verwendet werden, ausgewählt sind.

8. Zweiteilige kosmetische Zusammensetzung, umfassend:
- einen ersten Teil, der in einem kosmetisch unbedenklichen Medium ein oder mehrere Vinylformamid/Vinylformamin-Copolymere umfasst, welche Folgendes umfassen:
20 bis 40 Mol-% Einheiten der folgenden Formel A: 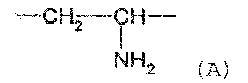 und 60 bis 80 Mol-% Einheiten der folgenden Formel B: 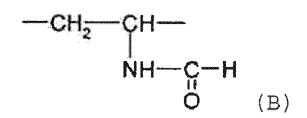 wobei das bzw. die Vinylformamid/Vinylformamin-Copolymere ausschließlich aus Einheiten der Formel A und Einheiten der Formel B aufgebaut ist bzw. sind,
und
- einen zweiten Teil, der in einem kosmetisch unbedenklichen Medium ein oder mehrere Assoziativverdicker, die von den Vinylformamid/Vinylformamin-Copolymeren verschieden sind, umfasst.

9. Verfahren zur kosmetischen Behandlung der Haare, **dadurch gekennzeichnet, dass** es darin besteht, dass man auf die Haare eine wirksame Menge einer Zusammensetzung nach einem der Ansprüche 1 bis 8 aufbringt und dann nach einer fakultativen Einwirkungszeit eine fakultative Spülung durchführt.

10. Verwendung einer Zusammensetzung nach einem der Ansprüche 1 bis 8 zum Gestalten und/oder Fixieren der Frisur.

## Claims

1. Cosmetic composition comprising, in a cosmetically acceptable medium:
- one or more vinyformamide/vinyformamine copolymers comprising:
from 20 to 40 mol% of units of following formula A: 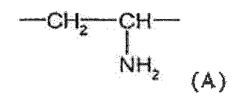 and from 60 to 80 mol% of units of following formula B: 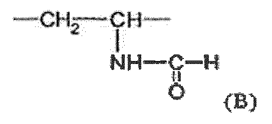
the vinyformamide/vinyformamine copolymer(s) being constituted solely of units of formula A and units of formula B,
and
- one or more associative thickening polymers other than the vinylformamide/vinylformamine copolymers.

2. Composition according to the preceding claim, **characterized in that** the vinyformamide/vinyformamine copolymer(s) are present in proportions ranging from 0.1 to 25% by weight, relative to the total weight of the composition.

3. Composition according to either one of the preceding claims, **characterized in that** the thickeners are present in the compositions in proportions ranging preferably from 0.01 to 20% by weight, more preferably from 0.05 to 5% by weight and more particularly from 1 to 4% by weight, relative to the total weight of the composition.

4. Composition according to any one of the preceding claims, **characterized in that** the weight ratio between the total amount of vinyformamide/vinyformamine copolymer(s) on the one hand and the total amount of thickener on the other hand is between 0.1 and 20, preferably between 1 and 15 and better still between 1 and 8.

5. Composition according to any one of the preceding claims, **characterized in that** it also comprises from 0.01 to 10% by weight, relative to the total weight of the composition, of amphoteric or zwitterionic surfactant(s).

6. Composition according to any one of the preceding claims, **characterized in that** it also comprises from 0.01 to 10% by weight, relative to the total weight of the composition, of cationic surfactant(s).

7. Composition according to any one of the preceding claims, **characterized in that** it also contains one or more additives chosen from pearlescent agents, opacifying agents, plasticizers, sunscreens, fragrances, dyes, preservatives, pH stabilizing agents, acids, bases, polyols, mineral fillers, glitter and any other additive conventionally used in the field of cosmetics.

8. Two-part cosmetic composition, comprising:
- a first part comprising, in a cosmetically acceptable medium, one or more vinyformamide/vinyformamine copolymers which comprises:
from 20 to 40 mol% of units of following formula A: 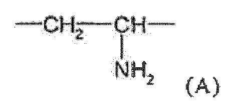 and from 60 to 80 mol% of units of following formula B: 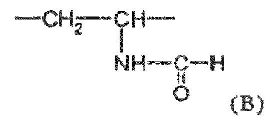
the vinyformamide/vinyformamine copolymer(s) being constituted solely of units of formula A and units of formula B,
and
- a second part comprising, in a cosmetically acceptable medium, one or more associative thickeners other than the vinylformamide/vinylformamine copolymers.

9. Method for the cosmetic treatment of the hair, **characterized in that** it consists in applying, to the hair, an effective amount of a composition according to any one of Claims 1 to 8, then in carrying out an optional rinsing step after an optional leave-in time.

10. Use of a composition according to any one of Claims 1 to 8, for hair shaping and/or fixing the hairstyle.
